# EUROPEAN PATENT SPECIFICATION

(11) **EP 1 720 909 B1**
(45) Date of publication and mention of the grant of the patent: **23.11.2011**
(21) Application number: 05723280.3
(22) Date of filing: 17.02.2005
(51) Int. Cl.: C07K 16/18, A61K 39/395, A61P 25/28, C12N 15/13

(54) **ANTI-ABETA ANTIBODY**
ANTI-ABETA ANTIKÖRPER
ANTICORPS ANTI-ABETA

(30) Priority: 23.02.2004 US 546764 P
(43) Date of publication of application: 15.11.2006
(73) Proprietor: ELI LILLY AND COMPANY, Indianapolis, IN 46285 (US)
(72) Inventor: DEMATTOS, Ronald, Bradley, Noblesville, IN 46060 (US); KUCHIBHOTLA, Uma, Indianapolis, IN 46236 (US); YANG, Hsiu-Chiung, Indianapolis, IN 46203 (US); MCCLURE, Don, B., Indianapolis, IN 46256 (US)
(74) Representative: Commander, Paul Martin Brial
(86) International application number: PCT/US2005/005198
(87) International publication number: WO 2005/082939

(56) References cited:
- WO-A-02/46237
- WO-A-02/088306
- YOO E M ET AL: "Myeloma expression systems" JOURNAL OF IMMUNOLOGICAL METHODS, ELSEVIER SCIENCE PUBLISHERS B.V.,AMSTERDAM, NL, vol. 261, no. 1-2, 1 March 2002 (2002-03-01), pages 1-20, XP004341264 ISSN: 0022-1759
- CHAUHAN NEELIMA B ET AL: "Intracerebroventricular passive immunization with anti-Abeta antibody in Tg2576." JOURNAL OF NEUROSCIENCE RESEARCH, vol. 74, no. 1, 1 October 2003 (2003-10-01), pages 142-147, XP002339442 ISSN: 0360-4012
- MILLER DAVID L ET AL: "Humoral immune response to fibrillar beta-amyloid peptide." BIOCHEMISTRY, vol. 42, no. 40, 14 October 2003 (2003-10-14), pages 11682-11692, XP002339443 ISSN: 0006-2960
- BARNES L M ET AL: "Advances in animal cell recombinant protein production: GS-NSO expression system" CYTOTECHNOLOGY, vol. 32, no. 2, February 2000 (2000-02), pages 109-123, XP002301322 ISSN: 0920-9069

## Description

### FIELD OF THE INVENTION

This invention is in the field of medicine. More particularly, this invention is directed to a process for preparing an anti-Aβ antibody either free of Aβ peptide or with acceptably low amounts thereof.

### BACKGROUND OF THE INVENTION

A principal component of amyloid plaques is the 39 to 43 amino acid Aβ peptide. This peptide is proteolytically derived from a type I integral membrane protein, the amyloid precursor protein (APP). The predominant forms secreted in cell culture media are Aβ peptide (1-39/40 or X-39/40), whereas the longer forms, Aβ peptide (1-42/43 or X-42/43), which are less soluble and more prone to aggregate, constitute the nucleating seeds for amyloid deposition. Amyloid deposits comprised of Aβ peptide (1-42/43) are associated with conditions and diseases such as Alzheimer's disease, Down's syndrome, cerebral amyloid angiopathy, vascular dementias, mild cognitive impairment, and the like.

Various therapeutic treatments for conditions and diseases related to abnormal deposits containing the Aβ peptide have focused on preventing Aβ peptide production and/or its aggregation into plaques as well as on reducing or eliminating amyloid plaques. Another treatment approach involves inducing an immunogenic response to Aβ peptide through administration of the peptide by, for example, active immunization (WO 99/27944). However, a recent Phase 2A study utilizing an active immunization approach with synthetic human Aβ 1-42 peptide was suspended as soon as four patients experienced clinical signs consistent with inflammation in the central nervous system (CNS). Since the suspension, eleven additional patients developed symptoms associated with CNS inflammation (Orgogozo et al., Neurology, 61:46-54 (2003); Schenk et al., Curr. Opn. Immun., 16:599-606 (2004)). As such, administration of Aβ peptide to treat Alzheimer's disease has caused adverse events and raised safety considerations for the patient.

An alternate immunological means for targeting Aβ peptide is through the administration of antibodies specific for the peptide by, for example, passive immunization. While passive immunization does not establish memory in T and B cells in the manner that active immunization does, the passive approach has not raised the safety concerns that surround active immunization.

WO 02/46237 relates to humanized antibodies that recognize beta amyloid peptide.

Previously, various cell lines, such as K562, M17, HEK 293, CHO, and HUVEC, were shown to produce Aβ peptide (Shoji, et al., Science, 258:126-129 (1992); Haass, et al., Nature, 359:322-325 (1992)). Accordingly, many cell lines that can be used to express human or humanized antibodies for clinical use, such as CHO and HEK 293, endogenously contain APP holoprotein as well as the γ- and β-secretases necessary to cleave APP and thereby naturally express Aβ peptide.

Surprisingly, during the preparation of anti-Aβ antibodies, it was discovered that Aβ peptide, endogenously produced in most mammalian cell lines commonly used to express antibodies recombinantly, binds to the expressed anti-Aβ antibody at low levels and is carried through the cell culture and purification process. Along with Aβ peptide contamination of recombinantly-produced anti-Aβ antibody material, there is a potential for an increased immunogenic response in a patient, making prevention, removal, or reduction of the Aβ peptide of key importance. Furthermore, when the endogenously produced Aβ peptide is non-human, as with a CHO cell line, the immunogenicity implications for non-human Aβ peptide bound to the expressed anti-Aβ antibody may cause even greater concern for patient safety and, thus, make prevention, removal, or reduction of the Aβ peptide vitally important.

### SUMMARY OF THE INVENTION

According to the present invention there is provided a process for preparing an Aβ antibody comprising the steps of:
a. expressing the antibody in cells that endogenously express Aβ peptide;
b. adding a beta or gamma secretase inhibitor to media used to grow the cells; and
c. purifying the antibody from the growth media wherein the purified antibody has an undetectable concentration or has acceptably low levels of endogenously produced Aβ peptide.

Preferably, a gamma secretase inhibitor is added to the media.

More preferably, the cells are mammalian cells and are hamster, human, or mouse cells, selected from CHO, HEK 293 and PER.C6 cells.

Another embodiment provides that the antibody is expressed in cells in which Aβ production is eliminated through deletion of a specific gene, such as that encoding APP, β-secretase, or one of the γ-secretase genes, or through increased expression of α-secretase. A further embodiment provides that the antibody is produced in a cell culture that contains α β- or γ-secretase inhibitor.

### DETAILED DESCRIPTION OF THE INVENTION

For the purposes of the present invention, as disclosed and claimed herein, the following terms are as defined below.

By "antibody" is meant a whole antibody, including without limitation a chimeric, humanized, human, recombinant, transgenic, grafted and single chain antibody, and the like, or any fusion proteins, conjugates, fragments, or derivatives thereof that contain one or more domains that selectively bind Aβ peptide. Antibody thereby includes a whole immunoglobulin molecule, a monoclonal antibody, a chimeric antibody, a humanized antibody, a human antibody, or an immunologically effective fragment of any of these. An antibody fragment means an Fv, a disulfide linked Fv, scFv, Fab, Fab', or F(ab')₂ fragment, which are well known in the art. The expression "anti-Aβ antibody" means an antibody that recognizes or binds Aβ peptide.

The term "humanized antibody" means an antibody that is composed partially or fully of amino acid sequences derived from a human antibody germline or a rearranged sequence and made by altering the sequence of an antibody having non-human complementarity determining regions (CDR). The framework regions of the variable regions are substituted by corresponding human framework regions leaving the non-human CDR substantially intact The human framework regions include genomic framework regions, and also encompasses those containing one or more amino acid substitutions. In particular, such substitutions include mutations in which an amino acid at a particular position in the human framework is replaced with the amino acid from the corresponding position of the natural framework for the non-human CDR. An antibody in the context of humanized antibody is not limited to a full-length antibody and can include fragments and single chain forms.

The term "Aβ peptide" or "Aβ" in this context includes the 39, 40, 41, 42, and 43 amino acid peptides derived from the amyloid precursor protein (APP) protein *in vivo* by proteolysis, and any fragments of those peptides, such as N-terminally shortened peptides derived from those peptides (e.g., denoted by, for example, x-42, where x =1, 2, 3, etc.), C-terminally shortened peptides derived from 1-39,40,41, and 42 peptides, and peptides shortened at both termini. See SEQ ID NO:1, human Aβ peptide and SEQ ID NO:2, rodent Aβ peptide (i.e. Mouse, Hamster) for details of each full-length amino acid peptide sequence.

As used herein, the term "β-secretase" refers to the enzyme involved in processing APP which cleaves APP to generate the amino terminus of Aβ peptide. The term "γ-secretase" refers to the enzyme complex involved in APP processing which cleaves APP subsequent to β-secretase to generate the carboxyl terminus of Aβ. The term "α-secretase" refers to the enzyme involved in APP processing which cleaves APP within the Aβ sequence (between Aβ peptide residues 16 and 17) in a pathway for soluble APP such that Aβ peptide is not produced. By "β- or γ-secretase inhibitors" is meant molecules which inhibit (block or reduce) β- or γ-secretase enzymatic activity

By "acceptably low levels of Aβ peptide" is meant a level of contaminating Aβ peptide in an anti-Aβ antibody preparation that would be deemed safe and thereby acceptable or suitable for administration to a human subject, particularly in a pharmaceutical composition. In particular, acceptably low levels of Aβ peptide would be those which would not cause an immunogenic response and/or an increased immunogenic response in a patient administered anti-Aβ antibody. Acceptably low levels would be determined by one of skill in the art following practices that are commonly used and accepted in the development of pharmaceutical compositions and formulations with respect to safety.

By "undetectable concentration" of Aβ peptide is meant a concentration of Aβ peptide that would fall below the detection limits of methods commonly used to measure the concentration of Aβ peptide in a preparation of anti-Aβ antibody. Such methods include, but are not limited to, ELISA, acid-urea gel/western blot analysis (as described in Examples 1-3), mass spectrometric methods, analytical chromatographic methods, or other highly sensitive analytical methods. For example, the acid gel/western analysis as described in Examples 1-3 has a maximum sensitivity of~1 pg Aβ/µg IgG, while the ELISA used in Example 3 has a limit of detection of 0.02 ng/mL. Concentrations of Aβ falling below these limits for these respective methods would be undetectable.

The compositions disclosed herein may be made by any of several methods known in the art. The following methods are intended to illustrate but not to limit the invention.

In general, recombinant antibody production is accomplished using processes that can be grouped into three major stages: cell line generation, cell culture, and purification. Thus, after a cell line is generated, an anti-Aβ antibody of the present invention is generally prepared by a process that includes expressing the antibody in a cell line and purifying the antibody. Changes made at any of these stages may impact expression or characteristics of the antibody generated. A number of variables can affect antibody expression at the cell line generation stage, including vector constructs and leader sequences contained therein used to transform the cell line to enable expression of the antibody, choice of cell type, selection of transfected cells, gene amplification and cell line screening. Antibody expression from the selected cell line relies upon the use of medium for cell culture. Media modifications such as changes to temperature, nutrients, and dissolved oxygen can impact expression and the product quality. Following antibody expression in cell culture, purification techniques such as various chromatographic techniques, filtration, and buffer exchange can alter the properties of the desired product, as well as purity and the nature of contaminants. In view of these general recombinant antibody production stages, the present invention can be achieved by using particular techniques or making specific modifications at each of these stages.

Processes for making compositions disclosed herein involve particular sources of the cell line as well as modifications to the cell line. As previously mentioned, the cell line affects antibody expression. Processes for making compositions disclosed herein include use of mammalian cell lines for expressing anti-Aβ antibody. Preferably, the mammalian cell line is a hamster, human, or mouse cell line. More preferably, the mammalian cell line is CHO, HEK 293 or PER.C6. Most preferably, the mammalian cell line is CHO .

Mammalian cell lines that lack APP or one of the secretases (β- or γ-secretase) can be used for expressing the recombinant antibodies. A cell line that lacks or has reduced levels of APP, β- or γ-secretase can be achieved through various cell line manipulations or modifications. Cell lines in which gene(s) encoding APP, β- or γ-secretase are knocked out can be generated by methods well known in the art. Alternatively, modifications to the cell line can produce this effect of lacking a gene. An example of a useful cell line modification involves significantly reducing the amount of Aβ peptide expressed by degrading the RNA transcript for the undesirable protein (e.g. APP or β- or γ-secretase) through a process known as RNA interference. To enable RNA interference, cells can be stably or transiently transfected, or infected, with a DNA sequence to provide plasmid or viral mediated expression of small hairpin RNA structures which specifically bind to the transcript of interest to initiate cleavage and degradation of that transcript according to methods known in the art (Banan and Puri, Curr. Pharm. Biotechnol., 5:441-50 (2004); Nesterova and Cho-Chung, Curr. Drug Targets, 5:683-9 (2004); Medema, Biochem J., 380:593-603, (2004)). As an alternative to mammalian cell culture, transgenic plants or plant cell cultures have been used for expression of proteins (Hellwig et al., 2004, Nature Biotechnology, 22:1415 (2004)), and may be another source for producing antibodies that lack Aβ. Likewise, various species of yeast are commonly used as an alternative to mammalian cell culture and could be applicable for expression of antibodies that lack Aβ. Use of these methods reduces or prevents production of Aβ peptide.

Methods for making compositions disclosed herein also involve modifications to the cell culture. These methods include incorporating β- or γ-secretase inhibitors in the cell culture to produce anti-Aβ antibody with acceptably low levels of Aβ peptide present. Various β- and γ-secretase inhibitors are known (e.g. U.S. Patent No. 6,486,350, U.S. Patent No. 6,627,739, Dovey et al., J Neurochem., 76:173-181 (2001); Yue-Ming et al., Nature, 405: 689-694 (2000)) and can be used for these methods.

Additional methods for making compositions disclosed herein increase soluble APP production in the cell culture, thereby reducing the amount of Aβ peptide produced. Soluble APP production may be increased through increased α-secretase activity in the cell line. A cell line with increased α-secretase activity can be generated by methods known in the art. Alternatively, soluble APP production is increased with copper addition to CHO cells (Borchardt et al., Biochem J., 344:431-467 (1999)). Copper addition also greatly reduced levels of Aβ peptide in parental CHO-K1 cells and in copper-resistant CHO-CUR3 cells.

Methods for making compositions disclosed herein also involve various purification techniques. These techniques include Protein A capture of antibody from cell culture. Subsequent purification may include use of agents to dissociate the anti-Aβ antibody from the Aβ peptide followed by separation of the antibody from antigen based on chromatographic differences between these two entities. Preferred dissociative agents include acid, urea, thiocyanate, and detergent. After achieving the dissociation of the antibody and the antigen, chromatographic techniques capable of separating dissociated anti-Aβ antibody from Aβ peptide are used to remove the antigen from the antibody or from the antibody-antigen complex. These chromatographic techniques are preferably size exclusion chromatography, ion-exchange chromatography, reverse phase chromatography, and hydrophobic interaction chromatography. Additionally, tangential flow filtration is used as another technique for separating the antigen from the antibody. In a preferred purification method, compositions disclosed herein are purified by steps that include Protein A capture, neutralization, dilution, acidification of the antibody, size exclusion chromatography, and neutralization (see Example 2).

Another chromatographic method uses an immobilized antibody to another epitope of Aβ peptide or an antibody with higher affinity to Aβ peptide to isolate and remove the antibody-antigen complex or the antigen.

The compositions disclosed herein may be used to treat human patients with conditions and diseases such as Alzheimer's disease, Down's syndrome, cerebral amyloid angiopathy, vascular dementias, mild cognitive impairment, and the like. Aβ peptide may raise potential immunogenicity risks for the patient, with even greater potential health concerns if the Aβ peptide is non-human. As such, prevention, removal or reduction of Aβ peptide is key.

The compositions disclosed herein are suitable for administration to a human subject in a pharmaceutical composition that includes an anti-Aβ antibody and a pharmaceutically acceptable excipient. Examples of acceptable excipients include buffers, surfactants, preservatives, solubilizing agents, isotonicity agents, stabilizing agents and the like designed to be appropriate for the selected mode of administration. Remington's Pharmaceutical Sciences, Mack Publishing Co., Easton PA, latest edition, provides a compendium of formulation techniques as are generally known to practitioners.

The following examples are intended to illustrate but not limit the invention.

### Example 1

### Expression of anti-Aβ peptide in cell culture containing a γ-secretase inhibitor.

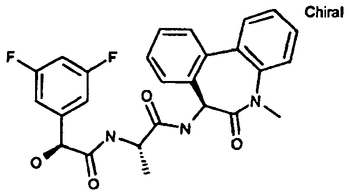

A γ-secretase inhibitor, (WO 98/28268), is added to the HEK 293 cell culture in which an anti- Aβ antibody is being expressed to reduce the amount of Aβ peptide naturally expressed by cells. Cell culture samples for this example include a control culture with no inhibitor added, a culture in which I nM inhibitor is added at time = 0 and every 24 hours thereafter for 5 days (for a 5 day transfection), and a culture in which 1 nM inhibitor is added at time = 0. These samples are purified using a Protein A column as well as size exclusion chromatography. The samples are analyzed for Aβ peptide content by acid-urea gel separation and subsequent western blot analysis as detailed below. Results of anti-Aβ produced and analyzed in this manner are present below in Table 1.

The following protocol describes a technique for formic acid denaturation of samples and subsequent electrophoresis through an acid-urea polyacrylamide matrix:

### Day 1

### Apparatus setup

1) Clean and assemble acrylamide gel plates for casting. For example, Hoeffer plates into a casting stand (16 cm x 14 cm plates with 1.5 mm spacers). Clean plates thoroughly with detergent, rinse in distilled H₂O (dH₂O), rinse with acetone, and finally rinse with 100% EtOH.
2) Mark plates at 10 cm (22% separating gel) and 11.75 cm (10% step gel).

### Making the Gel and Gel Concentrations

1) Acrylamide (40% solution, such as pre-made Bio-Rad/cat#161-0148)
2) Gel components:

| | 4% stacker | 10% | 22% |
|---|---|---|---|
| Urea | 5.76 g | 2.88 g | 11.56 g |
| 40% Acrylamide | 1.6 mL | 2 mL | 17.6 mL |
| GAA | 1.6 mL | 800 µL | 3.2 mL |
| TEMED (2.5%) | 450 µL | 200 µL | 600 µL |
| APS(10%) | 100 µL | 100 µL | 100 µL |
| dH₂O | to 16 mL | to 8 mL | to 32 mL |

Add urea, acrylamide, glacial acetic acid (GAA), and dH₂O into 50 mL conical tubes. Vortex briefly and incubate in a 55 °C water bath. Vortex every few minutes until the urea is completely in solution. Degas 22% and 10% solutions and place solutions on ice, leaving the 4% stacker in the water bath.

### Pouring the Gel (at room temperature)

1) Add N,N,N',N'-tetramethylethylenediamine (TEMED) to the 22% solution and gently invert several times to mix. Next add the 10% APS (ammonium persulfate) and invert several times. Using a 25 mL pipette, slowly pour solution between the plates until it reaches the 10 cm mark.
2) Carefully add a 750 µL overlay of 5% GAA. Use a P1000 pipette to very slowly let the 5% GAA run down one side of the glass plates onto the 22% resolving gel.
3) Allow to polymerize at room temperature for at least 1 hour.
4) Add the TEMED and 10% APS to the 10%-acrylamide solution in the same manner as for the 22%. Remove the overlay and add the solution until reaching the second mark at 11.75 cm. Again, carefully pour a 750 µL overlay of 5% GAA as above. Allow the gel to polymerize for 30 minutes.
5) Prior to adding the 4% stacker solution, place the gel stand in a 37 °C incubator for 15 minutes to warm gels. This aids in the wells polymerizing correctly. Degas the 4% stacker solution and keep at 55 °C. Remove the overlay. Add the TEMED, invert several times, and then add the 10% APS. Quickly pour this solution and insert a clean-dry 12-well comb (or a 15-well comb). After pouring the stacker, place the stand back in the incubator for 15 minutes then remove. Allow to polymerize on the bench top. The stacker will take at least 1.5 hours to polymerize completely.
*Note: During the polymerization steps, air pockets may appear in the resolving gel. These pockets form due to the change in temperature of the gel during and after polymerization. These spaces do not affect the performance of the technique.

### Sample Preparation

Analysis upwards of 3 mg of protein in a single lane will achieve reasonable band results. Conditions for analyzing protein are as follows.

### Sample:

30 µL of 100 mg/mL protein (maximum concentration of protein)
80 µL formic acid (98%) (ICN cat#15162-90)
20 µL Acid Loading Buffer (80% formic acid, 60% sucrose and 0.02% methyl green; Make buffer by dissolving 6 g sucrose in ~8 mL of ~99% formic acid. Heat and agitate the mixture. After the sucrose has dissolved, the volume of the solution is adjusted to 10 mL with ~99% formic acid. Add 2 µL of 1% methyl green solution.)
1µl β-Mercaptoethanol
   *Note: Adjust volumes as needed. However, ensure that the final formic acid concentration is always between 70% to 90%.

### Ladders:

Pharmacia molecular weight markers, M.W. Range 2,512-16,949 (cat#80-1129-83). Reconstitute protein in 1 mL PBS. Freeze 10 µL aliquots at -20°C. Thaw 1 aliquot for every ladder needed and add 90 µL of formic acid (98%), 20 µL of Acid Loading Buffer, and 1 µL of β-Mercaptoethanol.
*Note: Do not reconstitute these ladders according to manufacturer's instruction (because SDS will cause the ladder to smear).

### BSA samples:

Distortion occurs for the outside two lanes of every gel run. To help minimize the negative effects that this distortion creates, load BSA samples in the outside lanes on either side of the gel. BSA samples =1 µL of 5% BSA, 90 µL formic acid (98%), 20 µL Acid Loading Buffer, and 1 µL of β- Mercaptoethanol.

### Running the Gel

Pre-Run: Assemble the apparatus in a cold room. Fill the bottom chamber (three-fourths the volume) with pre-chilled Acid Gel Running Buffer. Add appropriate amount of buffer to the top reservoir. Pre-run the gel Anode to Cathode at 250 volts for 30 minutes.
*Remove any air bubbles at the bottom of the gel and verify that the buffer has not leaked from the top reservoir.
**Acid Gel Running Buffer = 250 mL glacial acetic acid + 3750 mL dH₂O

Load Samples: Rinse wells with buffer prior to loading samples to help remove excess urea. Load samples, ladders, and outside BSA samples. Note that two ladders are run so that prior to transfer, one lane containing a peptide ladder is cut and stained with Coomassie Blue.

Step-Voltage: Run the gel Anode to Cathode as follows:
■ 15 minutes at 25 volts
■ 15 minutes at 50 volts
■ 15 minutes at 100 volts
■ 15 minutes at 200 volts
■ ~ 15 hours at 275 volts

**Run the gel overnight until the dye front is ~ 2 to 2.5cm from the bottom, which is generally the next morning if the gel was started in the late afternoon.

### Day 2

### Transfer Conditions for the Acid Urea Gel (run in 4°C cold room)

The night before transfer, make the following buffer and store in the cold room.

| Transfer Buffer: | |
|---|---|
| Tris-Base | 12.36 g |
| Glycine | 57.6 g |
| Methanol | 800 mL |
| dH₂O | Up to 4 liters. |

Neutralize the acid-urea gel prior to transfer. Carefully remove the gel and place it in a washed glass tray. Add 200 mL of transfer buffer and rock gently for 15 minutes. Repeat this wash step a total of four times. Perform transfer as per normal (2.5 hours at 100 volts).

### Ponceau S Staining and Destaining

After transfer, visualize the proteins in the ladder by Ponceau-S staining. The nitrocellulose is stained for 5 minutes in a solution of 0.1% Pon-S in 5% acetic acid. After destaining the membrane with dH₂0 (three very short washes), digitally scan the membrane and mark the ladders with a dull pencil. Save the file.
*Note: This ladder is used as solely for alignment purposes. This technique separates peptides according to their molecular weight (size) and charge. For example, two peptides of the same molecular weight but with differing charges probably do not have the same net mobility. Because of these issues, always run Aβ peptide standards in at least one well, which enables accurate Aβ peptide identification.
**Important: All bands from the peptide standard ladder do not transfer. When the Coomassie Blue ladder is aligned with the Ponceau-S stained nitrocellulose, it is apparent that the 10.7 kDa and 6.2 kDa bands do not transfer.

### Western Blot Conditions

Boil nitrocellulose membrane in PBS for 5 minutes. Proceed with usual Western conditions.

### Blocking Step:

Block membrane in 5% Milk in 1X tris-buffered saline/0.125% Tween 20® (TBS/T) for 45 minutes at 50 mL volume.

### Primary Antibody

Use a selected number of anti-Aβ antibodies (e.g. 3) such that the binding epitopes of the selected antibodies bind to different regions on the Aβ peptide. Ensure that the selected antibodies also allow for standard visualization of at least 79 pg. Primary antibody solution is in 0.5% Milk TBS/T with, for example, 1:1000 dilution of the selected antibodies at 20 mL total volume. Leave in primary antibody overnight on a rocker.

### Day 3

### Wash Membrane

Wash the membrane 3X in 1% BSA in TBS/T for 10-15 minutes each.

### Secondary Antibody

Secondary antibody solution is in 1% Milk TBS/T with 1:6000 dilution of anti-mouse HRP (Catalog# 7076 from Cell Signaling) at 50 mL total volume. Leave membrane in secondary antibody for 3 hours.

After secondary antibody, wash the membrane 3X with TBS/T for 15 minutes each.

### Development

Place membrane in enhanced chemiluminescence (ECL) solution (Pierce Super Signal West Pico Catalog # 34080) for 5 minutes prior to development

The maximum sensitivity of this procedure is dependent upon the reagents used during the Western blotting procedure. For the example detailed above, the maximum sensitivity of this assay is ~1 pg/µg IgG. For the samples, the IgG concentration (mg/mL) is determined by measuring the absorbance at 280 nm and dividing that value by an extinction coefficient of 1.4.

Analyses for samples generated according to this example provided the following results:

**Table 1. Acid urea gel analysis of purified antibody from cells either with or without a γ-secretase inhibitor.**

| Samples | FL¹ (pg/µg IgG) | T1² (pg/µg IgG) | T2³ (pg/µg IgG) | Total hAβ40 (pg/µg IgG) |
|---|---|---|---|---|
| No inhibitor | 70 | 38 | 70 | 178 |
| Inhibitor every 24 hours for 5 days | 4 | 0 | 3 | 7 |
| Inhibitor at T=0 | 56 | 27 | 52 | 135 |

| | | | | |
|---|---|---|---|---|
| ¹Full length hAβ40 ²N-terminal truncated hAβ40, #1 ³N-terminal truncated hAβ40, #2 | | | | |

### Example 2

### Purification of anti-Aβ antibody by acid dissociation of antibody and Aβ peptide.

An anti-Aβ antibody is expressed from HEK 293 cells grown in cell culture. The antibody is purified by applying the culture medium to a Protein A-agarose column and is eluted with 100 mM glycine buffer, pH 3.1. The pool of fractions eluted from Protein A is adjusted to about pH 7.4 by adding a small volume of 1M Tris buffer, pH 8.0. This pool of eluted fractions is then adjusted to about pH 2 by diluting 1:1 into 1 M glycine, pH 2. After about 10 minutes incubation at room temperature, the acidified pool is subjected to size exclusion chromatography on a 26/60 Superdex 200 column (Amersham) using a mobile phase of 50 mM glycine, 150 mM NaCl, pH 2 at a flow rate of 30 cm/hr. The antibody eluted from the size exclusion column is neutralized by adding Tris buffer and is dialyzed against PBS at pH 7.4.

Denaturing acid/urea gradient polyacrylamide gel analyses (see Example 1 for further description of this technique) provide mass estimations of Aβ peptide in units of pg per µg IgG for samples generated according to this acid dissociation method of purification. The following results were obtained following purification of anti-Aβ antibodies using this acid dissociation purification:

**Table 2. Acid urea gel analysis of purified antibody from HEK 293 cells: size exclusion chromatography only (no acidification) or acidification and size exclusion chromatography.**

| Sample | FL¹ (pg/µg IgG) | T1² (pg/µg IgG) | T2³ (pg/µg IgG) | Total hAβ40 (pg/µg IgG) |
|---|---|---|---|---|
| HEK 293, No Acid, SEC | 39 | 0 | 0 | 39 |
| HEK 293, Acid and SEC | 11 | 0 | 0 | 11 |

| | | | | |
|---|---|---|---|---|
| ¹Full length hAβ40 ²N-terminal truncated hAβ40, #1 ³N-terminal truncated hAβ40, #2 | | | | |

### Example 3

### Expression of anti-Aβ antibody in NS0 cells.

An anti-Aβ antibody is expressed from NS0 cells grown in cell culture. The antibody is purified by applying the culture medium to a Protein A-agarose column and is eluted with 100 mM glycine buffer, pH 3.1. The pool of fractions eluted from Protein A is adjusted to about pH 7.4 by adding 1M Tris buffer, pH 8.0. This pool of eluted fractions is then diluted 1:1 with PBS and is subjected to size exclusion chromatography on a 26/60 Superdex 200 column (Amersham) using a mobile phase of PBS, 150 mM NaCl, pH 7.4 at a flow rate of 30 cm/hr. The antibody eluted from the size exclusion column is dialyzed against PBS at pH 7.4. Using denaturing acid/urea gradient polyacrylamide gel analysis, no Aβ peptide was detected in anti-Aβ antibodies produced by this method.

ELISA analysis is used to quantitate the concentration of Aβ peptide. Wells of a 96-well ELISA plate (Nunc MaxiSorp™ F96 or C96) are coated with anti-Aβ antibodies (e.g. 2 or more) - that will recognize epitopes outside of the central Aβ peptide (e.g. 17-25) binding region - at a concentration of, for example, 7.5 µg/mL of each antibody in a coating buffer overnight at refrigerated conditions. After aspirating the coating solution from the plates, wells are blocked with 300 µL/well of HBST/Blotto (0.25% w/v nonfat dry milk in HEPES-Buffered Saline (10 mM and 150 mM, respectively) with EDTA (3mM) and Tween 20® (0.5% w/v)) for 1 to 2 hours at room temperature and then are washed 1X with Washing Buffer (1X PBS with 0.1% v/v Tween 20®) and are aspirated. Samples containing the antibody are appropriately diluted in HBST/Blotto and are applied to ELISA plates (100 µL per well). Equivalent dilutions are spiked with an additional 0.4 ng/mL synthetic rodent Aβ peptide 1-40 to ensure accurate quantitation in the diluted sample matrix. As a standard, synthetic rodent Aβ peptide 1-40 along with purified anti-Aβ antibody (with < 1 ppm total rodent Aβ peptide) is used. Control samples containing antibody (for total Aβ peptide control) and synthetic rodent Aβ peptide 1-42 spiked into the antibody (for Aβ peptide 1-42 control) are also tested. The ELISA plate is incubated for 1 to 2 hours at room temperature. The wells are washed 4X with Washing Buffer and are aspirated. Then, 100 µL/well of diluted (1:10,000) donkey anti-human IgG - alkaline phosphatase conjugate (Jackson Immunoresearch, # 709-056-149) in HBST/Blotto is applied to each well. After incubating for 1 to 2 hours at room temperature, washing 4X with Washing Buffer, and aspirating the wells, 100 µl of 1.0 mg/mL pNPP substrate (Kirkegaard & Perry Laboratories, Inc., # 50-80-00) solution in 1X DEA buffer solution is added to each well. The ELISA plate is incubated at room temperature with periodic shaking. Absorbance is read at 405 nm using a microplate reader when the color develops sufficiently (usually 2.0 to 2.5 absorbance units) for the standards. The limit of detection is 0.02 ng/mL. The limit of quantitation is 0.1 ng/mL. Concentration determinations for the standards are determined by AAA analysis. For the samples, the IgG concentration (mg/mL) is determined by measuring the absorbance at 280 nm and dividing that value by an extinction coefficient of 1.4. Using this ELISA analysis on anti-Aβ antibodies expressed in NS0 cells as described above, no Aβ peptide was detected.

### Example 4

### Cation exchange reduction of Aβ peptide in a preparation of humanized anti-Aβ antibody

A humanized anti-Aβ antibody preparation is expressed in CHO cells. The antibody is purified by applying the culture medium to a Protein A-agarose column and is eluted with 100 mM glycine buffer, pH 3.1. The pool of fractions eluted from Protein A is adjusted to about pH 7.4 by adding 1M Tris buffer, pH 8.0. The antibody preparation contains 15-20 ppm hamster Aβ peptide, as determined by ELISA.

The antibody is further purified by cation exchange chromatography as follows. The starting antibody material is diafiltered against 50mM sodium acetate at pH 5.2 (5 volumes, using a 30k cut-off PES tangential-flow ultra filter) to decrease the conductivity in preparation for loading onto the cation exchange column. The diafiltered protein solution is then applied to a SP Sepharose High Performance (GE Healthcare) column (0.66 x 15cm, loaded at 15 mg of protein per mL of column volume) equilibrated in 50mM sodium acetate at pH 5.2. All operations are performed at room temperature and a linear flow rate of 115 cm/hr. After charging, the column is washed with 5 column volumes of 50mM sodium acetate at pH 5.2 and the antibody is eluted either stepwise (5 column volumes of 50mM sodium acetate, 135mM NaCl, pH 5.2) or with a linear 15 column volume gradient from 0 to 150mM NaCl in 50mM sodium acetate, pH 5.2. Fractions from the main peak are combined (to achieve a yield of approximately 90%). Anti-Aβ antibodies purified in this manner resulted in pools having lower Aβ peptide content than the starting material (10 ppm for the step eluted material and 9 ppm for the gradient eluted material).

### Example 5

### Immunopurification reduction of Aβ in anti-Aβ antibodies

An anti-Aβ antibody which binds in the central domain of human Aβ between amino acids 13-28 is expressed from HEK 293 cells grown in cell culture. Contaminating human Aβ peptide is separated from this antibody by immunopurification using a monoclonal antibody directed against the carboxyl terminus of Abeta 40, 2G3, coupled to agarose beads. The antibody preparation is rotated overnight with the 2G3 coupled beads at a 10:1 volume ratio. Following the overnight incubation, the agarose beads are pelleted to remove 2G3-Aβ complexes. ELISA is then used to determine the amount of Aβ peptide present in the anti-Aβ antibody before and after immunopurification.

Preparations of anti-Aβ antibodies immunopurified in this manner and analyzed by ELISA were found to have 10-25 pg Aβ/µg IgG prior to purification and no detectable Aβ following purification. The reduction in contaminating Aβ was confirmed by acid gel/western analysis as described in Example 1.

## Claims

1. A process for preparing an Aβ antibody comprising the steps of:
a. expressing the antibody in cells that endogenously express Aβ peptide;
b. adding a beta or gamma secretase inhibitor to media used to grow the cells; and
c. purifying the antibody from the growth media wherein the purified antibody has an undetectable concentration or has acceptably low levels of endogenously produced Aβ peptide.

2. The process of Claim 1 wherein a gamma secretase inhibitor is added to the media.

3. The process of Claim 1 or 2 wherein the cells are mammalian cells.

4. The process of Claim 1 or 2 wherein the cells are hamster, human, or mouse cells.

5. The process of Claim 1 or 2 wherein the cells are selected from the group consisting of CHO, HEK 293 and PER.C6 cells.

## Patentansprüche

1. Verfahren zum Herstellen eines Aβ-Antikörpers, das die folgenden Stufen umfasst:
a. Exprimieren des Antikörpers in Zellen, die Aß-Peptid endogen exprimieren;
b. Zugeben eines β- oder γ-Sekretaseinhibitors zu den Medien, die zum Züchten der Zellen verwendet werden; und
c. Reinigen des Antikörpers von den Wachstumsmedien, wobei der gereinigte Antikörper eine nicht nachweisbare Konzentration oder annehmbar niedrige Spiegel von endogen produziertem Aß-Peptid aufweist.

2. Verfahren nach Anspruch 1, wobei der γ-Sekretaseinhibitor zu den Medien zugegeben wird.

3. Verfahren nach Anspruch 1 oder 2, wobei die Zellen Säugerzellen sind.

4. Verfahren nach Anspruch 1 oder 2, wobei die Zellen Zellen von Hamster, Mensch oder Maus sind.

5. Verfahren nach Anspruch 1 oder 2, wobei die Zellen aus der Gruppe ausgewählt sind, die aus CHO-, HEK 293- und PER.C6-Zellen besteht.

## Revendications

1. Procédé de préparation d'un anticorps anti-Aß comprenant les étapes de :
a. expression de l'anticorps dans des cellules qui expriment de manière endogène le peptide Aß ;
b. addition d'un inhibiteur de bêta ou de gamma sécrétase au milieu utilisé pour la croissance des cellules ; et
c. purification de l'anticorps à partir du milieu de croissance, où l'anticorps purifié présente une concentration non détectable ou présente des niveaux acceptablement bas de peptide Aß produit de manière endogène.

2. Procédé selon la revendication 1, où un inhibiteur de gamma sécrétase est ajouté au milieu.

3. Procédé selon les revendications 1 ou 2, où les cellules sont des cellules de mammifère.

4. Procédé selon la revendication 1 ou 2, où les cellules sont des cellules de hamster, humaines ou de souris.

5. Procédé selon la revendication 1 ou 2, où les cellules sont choisies dans le groupe constitué par les cellules CHO, HEK293 et PER.C6.


### SEQUENCE LISTING

<110> Eli Lilly And Company
<120> Anti-Abeta-Antibody
<130> x16324
<150> 60/546,764
   <151> 2004-02-23
<160> 2
<170> PatentIn version 3.2
<210> 1
   <211> 43
   <212> PRT
   <213> homo sapiens
<400> 1
   Asp Ala Glu Phe Arg His Asp Ser Gly Tyr Glu Val His His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val Ile Ala Thr
           35                  40
<210> 2
   <211> 43
   <212> PRT
   <213> Hamster sp.
<400> 2
   Asp Ala Glu Phe Gly His Asp Ser Gly Phe Glu Val Arg His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val Ile Ala Thr
           35                  40